(19)

Europäisches
Patentamt
European
Patent Office
Office européen
des brevets

(11) **EP 2 135 621 A1**

(12) **EUROPEAN PATENT APPLICATION**

(43) Date of publication:
**23.12.2009 Bulletin 2009/52**

(51) Int Cl.:
*A61K 45/06* (2006.01)        *A61P 15/08* (2006.01)
*A23L 1/302* (2006.01)

(21) Application number: **08425428.3**

(22) Date of filing: **18.06.2008**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(71) Applicant: **Fasani, Roberto
24100 Bergamo (IT)**

(72) Inventor: **Fasani, Roberto
24100 Bergamo (IT)**

(74) Representative: **Dragotti, Gianfranco et al
Dragotti & Associati srl
Via Marina 6
20121 Milano (IT)**

(54) **Combined formulations for the treatment of the male infertility**

(57)    The present invention refers to a formulation constituted of two different formulations which can be administered separately, one containing arginine, vitamin A, vitamin E, vitamin A, vitamin B6, vitamin B9 (i.e. folic acid), vitamin B12, coenzyme Q10 and the other containing carnitine, arginine, vitamin C and zinc, as well as its use for treating male infertility.

EP 2 135 621 A1

**Description**

The present invention refers to a formulation composed of two different formulations which can be administered separately , one containing arginine vitamin A, vitamin E, vitamin B6, vitamin B9 (i.e. folic acid), vitamin B12, coenzyme Q10 and the other containing carnitine, arginine, vitamin C and zinc, for treating male infertility.

**DESCRIPTION**

Poor sperm quality not linked to ascertainable causes, the so-called idiopathic oligoasthenoteratozoospermia (the OAT syndrome), is responsible for 40-75% of male infertility cases. Up to date, various pharmacological treatments have been proposed for the OAT syndrome. However, as of date, a rigorous evaluation of the studies available in literature has recently led the authors of *EAU Guidelines on Infertility - 2007*, to the conclusion that the treatments currently known do not meet the therapeutic needs towards such pathology.

For example, as a matter of fact, studies carried out on GnRH show contradictory results while the ones carried out on FSH proved inefficient. In addition, the "kininogene-kinin" system activating drugs have not yet been proved efficient while the mast cell blockers and the antiestrogens show a very low efficiency.

Lastly, the hCG/hMG combination, the androgens, the bromocriptines and the alpha blockers are not deemed recommendable unless in cases of hypogonadism, in that they cause adverse reactions.

Furthermore, over the last years several studies have shown the importance of the ROS (reactive oxygen species) in the physiopathology of reproductions: as a matter of fact, it has been shown how the oxidative stress combined with high levels of ROS in the seminal fluid (and in particular, but not solely, during inflammatory states leading to a large number of leukocytes) causes a reduction of the motility of sperms and damage to the cellular DNA *(Agarwal et al, 2006; Agarwal et al, 2002)*.

Sperms are very sensitive to the ROS given that their cellular membrane contains polyunsaturated fatty acid (PUFA) which are easily subjected to a peroxidation process *(Fraczek et al, 2007; Agarwal et al, 2005)*. Thus, the cellular damage would occur when the ROS production exceeds the activity of antioxidant mechanisms usually present in the seminal plasma *(Sheweita et al, 2005)*.

Various studies have been carried out over the last years regarding various compounds with antioxidant and spermatogenesis adjuvant action in infertile subjects allowing to draw important conclusions regarding the antioxidants:

- selenium and vitamin E, alone or combined, produce a reduction of the oxidative mechanisms and an improvement of the motility of sperms *(Keskes-Ammar et al, 2003; Scott et al, 1998)*;

- vitamin C is capable of improving the count, the motility and the morphology of the sperms *(Akmal et al, 2006)*;

- combination of vitamin C and E leads to a reduction of the damage of the DNA *(Greco et al, 2005)*;

- combination of L-carnitine and acetyl-L-carnitine proved efficient in improving the progressive sperm motility, in increasing the total antioxidant capacity of the seminal plasma and in increasing the pregnancy rates in males with OAT-syndrome *(Li et al, 2005; Balercia et al, 2005; Lenzi et al, 2004)*, even though a clinically or substantially relevant effect on the motility or on the total sperm count of mobile sperms in males affected by idiopathic asthenospermia is often denied *(Sigman et al, 2006)*;

and, regarding other compounds with an adjuvant action:

- folic acid and zinc sulphate, separately, lead to a substantial improvement of the sperm count in subfertile and fertile subjects *(Ebisch et al, 2006; Wong et al, 2002)*;

- in vitro addition of L-arginine and polyamines to sperms obtained from males with asthenospermia proved beneficial to the sperm motility *(Morales et al, 2003)*;

- vitamin B12, at high doses and with a long term treatment (16 weeks), is of great use in patients affected by oligozoospermia *(Moriyama et al, 1987)*;

- treatment of subjects affected by various degrees of oligoastenospermia with arginine opened important perspectives regarding the fertilising capacity of the latter *(Aydin et al, 1995)*.

- levels of coenzyme-Q10 in the seminal plasma were correlated to the sperm count and the motility *(Mancini et al, 1994)*.

An object of the present invention is a formulation constituted of two different formulations which can be administered separately, one containing arginine, vitamin A, vitamin E, vitamin A, vitamin B6, vitamin B9 (i.e. folic acid), vitamin B12, coenzyme Q 10 and the other containing carnitine, arginine, vitamin C and zinc. As a matter of fact, such formulation proved useful for the treatment of male infertility, in particular for the treatment of oligoasthenoteratozoospermia (OAT-syndrome).

Preferably, the abovementioned first formulation contains 300 to 500 parts by weight of arginine, 350 to 550 parts by weight of vitamin A, 1 to 3 parts by weight of vitamin B6, 0.0005 to 0.002 parts by weight of vitamin B12, 0.1 to 0.3 parts by weight of vitamin B9, 10 to 30 parts by weight of coenzyme Q10 and 0.07 to 0. 009 parts by weight of selenium while said second formulation contains 100 to 300 parts by weight of carnitine, 100 to 300 parts by weight of arginine, 0.05 to 0.15 parts by weight of vitamin C and 10 to 20 parts by weight of zinc.

More preferably, said first formulation contains 400 mg of arginine, 450 mg of vitamin A, 30 mg of vitamin E, 2 mg of vitamin B6, 0.001 mg of vitamin B12, 0.2 mg of vitamin B9, 20 mg of coenzyme Q10. 0.082,5 mg of selenium and said second formulation contains 200 mg of carnitine, 200 mg of arginine, 100 mg of vitamin C, 15.2 mg of zinc.

In addition, the formulation of the present invention may further contain common excipients and/or adjuvants such as, for example bulking, anti-caking, bonding and/or colouring agents. In particular, the formulation of the present invention comprises microcrystalline cellulose as a bulking agent, magnesium stearate and/or silicon dioxide as anti-caking agents and chlorophyll and/or curcuma as colouring agents.

According to the preferred implementation of the invention, the two abovementioned formulations are administered at time intervals in the range of 8 to 14 hours one from the other, preferably in the range of 10 to 12 hours.

In particular, said first formulation is administered in the morning, preferably before breakfast, while the second formulation is administered in the evening, preferably after dinner.

Said formulations are administered for a period of time in the range of 4 to 8 months, preferably for 6 months.

According to a further implementation of the invention, the abovementioned formulation is a combined formulation for simultaneous administration, separated or sequential, capable of substantially improving the sperm quality of patients affected by the OAT-syndrome.

The formulations according to the present invention are preferably indicated for oral administration, more preferably in form of a tablet, capsule or granule.

Further object of the present invention is the use of two different formulations which can be administered separately, the first containing arginine, vitamin A, vitamin E, vitamin B6, vitamin B9 (i.e folic acid), vitamin B12, coenzyme Q10 and the second containing carnitine, arginine, vitamin C and zinc for treating male infertility, in particular for treating idiopathic oligoasthenoteratozoospermia (OAT-syndrome).

In particular, the two abovementioned formulations are administered at a time interval in the range of 8 to 14 hours one from the other, preferably in the range of 10 to 12 hours.

In particular, said first formulation is administered in the morning, preferably before breakfast, while said second formulation is administered in the evening, preferably after dinner.

Said formulations are administered for a period of time in the range of 4 to 8 months, preferably for 6 months.

EXAMPLES

Example 1

Composition in tablet form to be taken every morning, before breakfast

    arginine 400 mg
    vitamin A 450 mg
    vitamin E 30 mg
    vitamin B6 2 mg
    vitamin B12 0.0011 mg
    vitamin B9 (folic acid) 0.2 mg
    coenzyme Q10 20 mg
    selenium 0.0825 mg
    microcrystalline cellulose 135.52 mg
    magnesium stearate 15 mg
    silicon dioxide 8 mg
    curcuma 5 mg

Example 2:

Composition in tablet form to be taken every evening, after dinner

    carnitine 200 mg
    arginine 200 mg
    vitamin C 100 mg
    zinc 15.2 mg
    microcrystalline cellulose 106 mg
    magnesium stearate 20 mg
    silicon dioxide 10 mg
    chlorophyll 2 mg

EXPERIMENTAL PART

Various infertile couples were recruited and subjected to the following preliminary tests

- two sperm tests, carried out in the same Laboratory, at a distance of about 2 ½ months one from the other, with a period of sexual abstinence 4-5 days ahead and a complete collection of the ejaculate completed by urethral expression from the perineum to the meatus, during a period of good health of the subject over at least the previous two months. Each sperm sample was examined, calculating its following parameters according to the *WHO - 1999* standards: ejaculate volume (ml), sperm concentration/mm$^3$, total motility (%) at 45', at 120' and at 180' (respectively partitioned into: rapid progressive (%), slow progressive (%), local (%), absent (%)), normal morphology (%).
- the Sperm Index (SI) of each sperm sample was calculated, with the aim of facilitating statistical investigation, according to the following formula:

$$SI = \frac{V \times C \times (mot\ a + \sqrt{mot\ b}) \times N}{10^{10}}$$

wherein "V" is the ejaculate volume (ml), "C" is the sperm concentration/ml, "mot a" is the rapid progressive motility (%), "mot b" is the slow progressive motility (%), "N" are the normal shapes (%). In brief, the SI attempts to represent in a single numerical value the total count of sperms well mobile and normal-shaped in an ejaculate. For example, an ejaculate having every parameter at its minimum normality value according to *WHO - 1999*. V = 2 ml; C = 20.000.000 sperms/ml; mot a = 25%; mot b = 25%; normal shapes = 15%) has an SI = 1.8, however a truly normal sperm having an SI ≥ 5.0.

- In addition, the following additional tests were performed on each semen sample: a) an aniline blue test, to evaluate the condensation and the maturity of the sperm chromatin (v.n.: < 50%) *(Terquem & Dadoune, 1983)*;

  b) the acridine orange test, which calculates the damage to the helices of the DNA of the sperms (v.n.: < 30%) *(Tejada et al, 1984)*;
  c) the swelling test, which identifies the functionally abnormal sperms, that is the ones with a lower fertilising capacity, as well as a higher rate of sperm implantation failure (v.n.: > 60%) *(Check, 2006)*;
  d) ARIC test, which evaluates the capacity of the sperms to penetrate into the oocytes (v.n.: > 14%.) *(Cummins et al, 1991)*.

- Scrotal Colour Doppler, to identify significant pathologies of the testicular pulp, lesions of the epididymis potentially suspected for obstructions and the presence of continuous venous refluxes in orthostatism along the internal spermatic veins.
- Transrectal ultrasonography, to identify significant pathologies of the prostate such as benign prostatic hyperplasia or suspicious cancerous lesions, and suspicious signs for (sub-)obstructions and/or calculosis of the ejaculatory ducts and problems regarding the ampulla-vescicular emptying.
- Karyotype

Furthermore, the following further tests were performed:

- cytology and culture test of the prostatic massage secretion *(Colpi et al, 1982)*, after 7 days of abstinence, only regarding patients whose anamnestic or objective data or whose transrectal ultrasonography data showed or raised suspicions regarding an inflammatory/infective pathology of the prostate.
- plasmatic FSH, LH, Testosterone, Prolactin, Estradiol only for patients with a sperm concentration below 20.000 sperms/mm$^3$.
- Search for Yq microdeletions, only on patients with a sperm concentration below 5.000 nemasperms/mm$^3$.

Inclusion criteria were considered in the present study:

- SI< 5;
- absence of significant pathologies according to Scrotal colour doppler (see above);
- absence of significant pathologies according to transrectal ultrasonography (see above);
- a normal karyotype and a negative search for Yq microdeletions , in cases requiring such test (see above);
- a negative cytology test on the prostatic secretion, or a positive cytology test (but with a negative culture test) after 2 weeks from the end of an antibiotic therapy which had lasted for at least 30 days;
- normal testosterone, prolactin and estradiol, in cases where such tests were required (see above);
- FSH not below the normality range, in cases where its performance was required (see above), in that a low value would have created grounds for a hormonal therapy.

As a whole 40 randomized patients were recruited, to whom the composition of example 1 was administered each morning and the composition of example 2 was administered each evening for a period of 6 months.
At the end of the treatment, all the patients were invited to be subjected to a new sperm test, according to the methods outlined above.

RESULTS

The treatment was well tolerated and there were no symptoms linked to the therapy observed on any of the patients.
The sperm volume increased considerably from 4.07 ± 1.83 ml (X±SD) before treatment to 4.55 ± 1.86 ml after treatment (p < 0.001).
The sperm concentration increased in a very significant manner from 26525 ± 25251 mm3 (X±SD) before treatment to 34200 ± 28254 after treatment (p < 0.0001).
The Total Sperm Motility increased from 47.40 ± 9.125 % (X±SD) before treatment to 51.08 ± 11.711 % after treatment at 45' (p = 0.011), from 44.10 ± 9.092 % to 47.28 ± 10.238 % at 120' (p = 0.018), and from 39.95 ± 9.546 % to 44.90 ± 11.600 % at 180' (p = 0.003) (Wilcoxon's test ).
Sperms with a Normal Morphology rose significantly from 8.18 ± 4.454 % (X±SD) before treatment to 10.15 ± 4.828 % after treatment (p = 0.001) (Wilcoxon's test ).
The Sperm Index improved significantly, passing from 0.820 ± 1.015 (X±SD) before treatment to 1.52±1.760 after treatment (p < 0.001) (two-tail test).
Sperms with positive Swelling Tests (on the 38 patients tested) did not vary significantly, from 66.18 ± 11.392 % (X±SD) before treatment to 67.11 ± 12.944 % after treatment (p = 0.34).

Sperms with positive ARIC Test (on the 34 patients tested) increased in a very significant manner from 7.71 ± 3.928 % (X±SD) before treatment to 10.15 ± 4.279 % after treatment (p < 0.001).

Sperms with chromatin maturation abnormalities detected by the Aniline Blue Test (on the 39 patients tested) did not vary significantly, passing from 48.51 ± 15.841 % (X±SD) before treatment to 45.72 ± 15.970 % after treatment (p = 0.113).

Sperms with DNA abnormalities detected by the acridine orange test (on the 36 patients tested) dropped significantly from 31.03 ± 13.185 % (X±SD) before treatment to 25.36 ± 14.137 after treatment (p = 0.013).

The formulation according to the present invention was therefore effective at significantly improving all the single parameters evaluated in a standard sperm test, also including the Sperm Index, which has the advantage of summarising the total count of well mobile and normally shaped sperms present in an ejaculate in a single numerical value.

Particularly important is the significant improvement induced by the therapy on the percentage of pathological sperms upon the Acridine Orange Test, especially nowadays, where the integrity of the DNA of the sperm is highly considered, with the aim of obtaining a pregnancy at term, both spontaneous and as a result of assisted reproduction techniques.

Equally relevant is the significant improvement of the percentage of sperms positive to the ARIC test, sign of an improved capacity of the sperm to fertilise an oocyte, such data being significant both when attempting to obtain spontaneous fertilisation, and when searching for a correct indication to use intrauterine insemination.

In conclusion, in the field of male infertility therapy, which nowadays shows an almost systematic use of the assisted reproduction techniques and specifically the ICSI, it appears quite interesting to be able to obtain significant sperm improvements using, in patients affected by an actual idiopathic oligoasthenoteratozoospermia (in that preliminarily well selected depending on the possible causal factors of their dispermy), a therapy with vitamins and minerals, such therapy being simple and safe, in that it is substantially free of contraindications.

**Claims**

1. Formulation constituted of two different formulations which can be administered separately, the first containing arginine, vitamin A, vitamin E, vitamin B6, vitamin B9, vitamin B12, coenzyme Q10 and the second containing carnitine, arginine, vitamin C and zinc.

2. Formulation according to claim 1, wherein said first formulation contains 300 to 500 parts by weight of arginine, 350 to 550 parts by weight of vitamin A, 1 to 3 parts by weight of vitamin B6, 0.0005 to 0.002 parts by weight of vitamin B12, 0.1 to 0.3 parts by weight of vitamin B9, 10 to 30 parts by weight of coenzyme Q10 and 0.07 to 0.09 parts by weight of selenium and said second formulation contains 100 to 300 parts by weight of carnitine, 100 to 300 parts by weight of arginine, 0.05 to 0.15 parts by weight of vitamin C and 10 to 20 parts by weight of zinc.

3. Formulation according to claims 1 and 2, wherein said first formulation contains about 400 mg of arginine, about 450 mg of vitamin A, about 30 mg of vitamin E, about 2 mg of vitamin B6, about 0.001 mg of vitamin B12, about 0.2 mg of vitamin B9, about 20 mg of coenzyme Q10 and about 0.0825 mg of selenium and said second formulation contains about 200 mg carnitine, about 200 mg arginine, about 100 mg vitamin C and about 15.2 mg zinc.

4. Formulation according to anyone of the preceding claims further containing excipients and/or adjuvants.

5. Formulation according to anyone of the preceding claims wherein said formulations are indicated for oral administration.

6. Formulation according to anyone of the preceding claims wherein said formulations are in form of a tablet, capsule or granule.

7. Formulation according to anyone of the preceding claims for use in treating male infertility.

8. Formulation according to claim 7 for the treatment of idiopathic oligoasthenoteratozoospermia.

9. Formulation according to anyone of the preceding claims wherein said formulations are administered at a time interval in the range of 8 to 14 hours.

10. Formulation according to claim 9 wherein said formulations are administered at a time interval in the range of 10 to 12 hours.

11. Formulation according to anyone of the preceding claims wherein said first formulation is administered in the morning and said second formulation is administered in the evening.

12. Formulation according to anyone of the preceding claims wherein said first formulation is administered before breakfast and said second formulation is administered after dinner.

13. Formulation according to anyone of the preceding

**EP 2 135 621 A1**

claims wherein the administration occurs for a period of time in the range of 4 to 8 months.

**14.** Formulation according to claim 13 wherein the administration occurs for a period of time of 6 months.

Europäisches
Patentamt

European
Patent Office

Office européen
des brevets

## EUROPEAN SEARCH REPORT

**Application Number**

EP 08 42 5428

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/032751 A (FASSI ALDO [IT]) 24 April 2003 (2003-04-24) * page 1, paragraphs 4,5 * * page 8, paragraph 1-3; claims 15,16 * | 1-14 | INV. A61K45/06 A61P15/08 A23L1/302 |
| X | WO 2007/087667 A (ANNERL BRIGITTE [AT] FAPA VITAL ANSTALT [LI]; ANNERL BRIGITTE [AT]) 9 August 2007 (2007-08-09) * page 2, lines 1-26 * * page 5, lines 10-18; table 1 * * page 10, lines 15-29; claims 9-11; table 3 * | 1-14 | |
| X | WO 03/086080 A (DAILY WELLNES COMPANY [US]) 23 October 2003 (2003-10-23) * page 2, paragraph 4; claims 21-35 * | 1-14 | |
| A | NIEDERBERGER ET AL: "Antioxidant Intake is Associated With Semen Quality in Healthy Men" JOURNAL OF UROLOGY, BALTIMORE, MD, US, vol. 174, no. 6, 1 December 2005 (2005-12-01), page 2301, XP005384177 ISSN: 0022-5347 * abstract; table III * | 1-14 | TECHNICAL FIELDS SEARCHED (IPC) A61P A61K A23L |
| X | US 2004/001817 A1 (GIAMPAPA VINCENT C [US]) 1 January 2004 (2004-01-01) * paragraph [0252] - paragraph [0416] * | 1-6,9-14 | |
| A | US 6 471 969 B1 (SCHLACHTER HERBERT [DE] ET AL) 29 October 2002 (2002-10-29) * claims * | 1-14 | |
| A | WO 02/076436 A (MEIJER JAAP [NL]) 3 October 2002 (2002-10-03) * claims * | 1-14 | |

The present search report has been drawn up for all claims

| Place of search | Date of completion of the search | Examiner |
|---|---|---|
| Munich | 6 November 2008 | Escolar Blasco, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 42 5428

This annex lists the patent family members relating to the patent documents cited in the above-mentioned European search report.
The members are as contained in the European Patent Office EDP file on
The European Patent Office is in no way liable for these particulars which are merely given for the purpose of information.

06-11-2008

| Patent document cited in search report | | Publication date | Patent family member(s) | | Publication date |
|---|---|---|---|---|---|
| WO 03032751 | A | 24-04-2003 | IT | RM20010621 A1 | 18-04-2003 |
| WO 2007087667 | A | 09-08-2007 | AT | 503219 A1 | 15-08-2007 |
| | | | CZ | 18013 U1 | 21-11-2007 |
| | | | DE | 212007000001 U1 | 30-08-2007 |
| | | | EP | 1978949 A2 | 15-10-2008 |
| WO 03086080 | A | 23-10-2003 | AU | 2002258727 A1 | 27-10-2003 |
| | | | CA | 2481603 A1 | 23-10-2003 |
| | | | EP | 1496747 A1 | 19-01-2005 |
| | | | JP | 2005522486 T | 28-07-2005 |
| US 2004001817 | A1 | 01-01-2004 | NONE | | |
| US 6471969 | B1 | 29-10-2002 | AT | 185949 T | 15-11-1999 |
| | | | AU | 6787896 A | 26-02-1997 |
| | | | BG | 102279 A | 30-12-1998 |
| | | | BR | 9609739 A | 21-12-1999 |
| | | | CA | 2228049 A1 | 13-02-1997 |
| | | | CN | 1193894 A | 23-09-1998 |
| | | | CZ | 9800269 A3 | 17-06-1998 |
| | | | DE | 59507140 D1 | 02-12-1999 |
| | | | DK | 755633 T3 | 17-04-2000 |
| | | | EA | 980175 A1 | 29-10-1998 |
| | | | EE | 9800033 A | 17-08-1998 |
| | | | WO | 9704668 A2 | 13-02-1997 |
| | | | EP | 0755633 A1 | 29-01-1997 |
| | | | ES | 2140589 T3 | 01-03-2000 |
| | | | FI | 980173 A | 24-03-1998 |
| | | | GR | 3032499 T3 | 31-05-2000 |
| | | | HK | 1015636 A1 | 01-04-2005 |
| | | | HU | 9900522 A2 | 28-06-1999 |
| | | | IL | 123066 A | 19-03-2001 |
| | | | IS | 4659 A | 26-01-1998 |
| | | | JP | 3512198 B2 | 29-03-2004 |
| | | | JP | 11510048 T | 07-09-1999 |
| | | | LT | 98025 A | 25-06-1998 |
| | | | LV | 12161 A | 20-11-1998 |
| | | | NO | 980358 A | 17-03-1998 |
| | | | PL | 324752 A1 | 08-06-1998 |
| | | | PT | 755633 T | 28-04-2000 |
| | | | SI | 9620105 A | 31-10-1998 |
| | | | SK | 12398 A3 | 05-08-1998 |
| | | | TR | 9800150 T1 | 21-04-1998 |
| WO 02076436 | A | 03-10-2002 | EP | 1469746 A2 | 27-10-2004 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 08 42 5428

This annex lists the patent family members relating to the patent documents cited in the above-mentioned European search report.
The members are as contained in the European Patent Office EDP file on
The European Patent Office is in no way liable for these particulars which are merely given for the purpose of information.

06-11-2008

| Patent document cited in search report | Publication date | Patent family member(s) | Publication date |
|---|---|---|---|
| WO 02076436 A | | NL 1017707 C2 | 01-10-2002 |

EPO FORM P0459

For more details about this annex : see Official Journal of the European Patent Office, No. 12/82